Europäisches Patentamt

European Patent Office

Office européen des brevets

(11) Publication number: **0 514 940 B1**

# EUROPEAN PATENT SPECIFICATION

(45) Date of publication of patent specification: **01.03.95**     (51) Int. Cl.⁶: **C07K 5/06**

(21) Application number: **92108708.6**

(22) Date of filing: **22.05.92**

(54) **Method of crystallizing alpha-L-aspartyl-L-phenylalanine methyl ester.**

(30) Priority: **23.05.91 JP 221336/91**
  **22.04.92 JP 102917/92**

(43) Date of publication of application:
  **25.11.92 Bulletin  92/48**

(45) Publication of the grant of the patent:
  **01.03.95 Bulletin  95/09**

(84) Designated Contracting States:
  **BE CH DE FR GB IT LI NL**

(56) References cited:
  **EP-A- 119 837**
  **JP-A-03 204 895**

  **CHEMICAL ABSTRACTS, vol. 116, no. 1, 6 January 1992, Columbus, Ohio, US; abstract no. 5558M, A YAZAKI ET AL.: 'solubility-improved aspartame granules' page 556 ;column LEFT ;**

(73) Proprietor: **Ajinomoto Co., Inc.**
  **No. 15-1, Kyobashi 1-chome**
  **Chuo-ku**
  **Tokyo (JP)**

(72) Inventor: **Abe, So, c/o Cent.Res.Lab.**
  **Ajinomoto Co.,Ltd.,**
  **No.1-1 Suzuki-cho,**
  **Kawasaki-ku**
  **Kawasaki-shi,**
  **Kanagawa-ken (JP)**
  Inventor: **Kishimoto, Shinichi, c/o Cent.Res.Lab.**
  **Ajinomoto Co.,Ltd.,**
  **No.1-1 Suzuki-cho,**
  **Kawasaki-ku**
  **Kawasaki-shi,**
  **Kanagawa-ken (JP)**
  Inventor: **Kato, Toshihisa, c/o Cent.Res.Lab.**
  **Ajinomoto Co.,Ltd.,**
  **No.1-1 Suzuki-cho,**
  **Kawasaki-ku**
  **Kawasaki-shi,**
  **Kanagawa-ken (JP)**
  Inventor: **Takeda, Hideo, c/o Cent.Res.Lab.**
  **Ajinomoto Co.,Ltd.,**
  **No.1-1 Suzuki-cho,**
  **Kawasaki-ku**
  **Kawasaki-shi,**
  **Kanagawa-ken (JP)**

(74) Representative: **Strehl Schübel-Hopf Groening & Partner**
  **Maximilianstrasse 54**
  **D-80538 München (DE)**

## Description

The present invention relates to a method of preparing α-L-aspartyl-L-phenylalanine methyl ester (hereinafter referred to as "α-APM") which is useful as a sweetener and relates to an improved method of crystallizing α-APM from a solution of α-APM.

α-APM of the present invention is a dipeptide sweetener having a sweetness about 200 times that of sucrose (cane sugar). Because of its good sweetening properties and the low calory content, it is widely used as a diet sweetener, and the worldwide demand for it is estimated to be over 10,000 tons by 1995.

α-APM is produced industrially by several methods. One method of obtaining α-APM is to react a N-substituted aspartic acid anhydride and a phenylalanine methyl ester in an organic solvent followed by removal of the N-substituent from the product by a conventional method (U.S. Patent 3,786,039). The thus formed α-APM containing impurities is brought into contact with a hydrohalogenic acid to obtain α-APM hydrohalide which is neutralized to obtain α-APM. A second method of obtaining α-APM is to methyl-esterify α-L-aspartyl-L-phenylalanine in a mixed solvent comprising water, methanol and hydrochloric acid to obtain α-APM hydrochloride, and then neutralize the hydrochloride to obtain α-APM (Japanese Patent Application Laid-Open No. 53-82752). A third method of obtaining α-APM is to condense an N-substituted aspartic acid and a phenylalanine methyl ester in the presence of an enzyme and thereafter remove the N-substituent from the product (Japanese Patent Application Laid-Open No. 55-135595).

In all of the above-mentioned methods, α-APM is finally crystallized by cooling from an aqueous solution having a relatively high temperature, then the resulting α-APM crystals are separated and dewatered in a solid-liquid separator, such as a centrifugal separator, and thereafter the thus separated crystals are dried. The cooling crystallization is generally carried out in a stirring crystallizer having a heat transfer surface for cooling or in a crystallizer equipped with an external heat exchanger and an external circulation system, such as pumps.

When crystallization of α-APM is carried out by cooling an α-APM-containing solution in a conventional forced flow system crystallizer with a stirring means or external circulation means, the solution always gives fine needle-like α-APM crystals having poor filterability and dewaterability. In addition, in such a system, numerous crystals precipitate and consolidate on the cooling heat transfer surface, whereby the heat transfer efficiency is markedly lowered. Therefore, to remove the consolidated crystals ("scale"), the operation of the crystallizer must be frequently stopped.

As a means of overcoming this problem, there is known a method of cooling an aqueous solution of α-APM by conductive heat transfer without effecting any forced flow such as mechanical stirring, to form a pseudo solid phase, optionally followed by further cooling the system (Japanese Patent Application Laid-Open No. 58-177952). In accordance with this method, peeling of the crystals from the cooling surface is extremely easy when discharging the crystal layer (sherbet-like pseudo solid phase);
and α-APM crystals having improved filterability and dewaterability in the successive solid-liquid separation step can be obtained. However, where existing or widely-used crystallization equipment is employed, the cooling takes a long time, the efficiency of the method is poor. The discharge causes frequent problems when the method is used with a conventional tank type crystallizer.

A need continues to exist for a means of overcoming the above-mentioned problems in the α-APM crystallization step, i.e., preventing the crystals from forming on the heat transfer surface, decreasing investment in equipment by using an existing or widely-used crystallizer and improving the solid-liquid separability of the crystal slurry.

## SUMMARY OF THE INVENTION

Accordingly, one object of the present invention is to provide a process which overcomes the problem of crystal formation on the cooling surface of an α-APM crystallization system (crystallizer).

This and other objects which will become apparent from the following specification have been achieved by the present process. In the present process, when α-APM is crystallized from its aqueous solution the pressure in the crystallizer is kept at or below atmospheric pressure (101 kPa 760 Torr) so as to concentrate the solution by vaporizing the solvent, whereupon the solution is cooled by the latent heat of vaporization and is thereby crystallized.

## BRIEF DESCRIPTION OF THE DRAWING

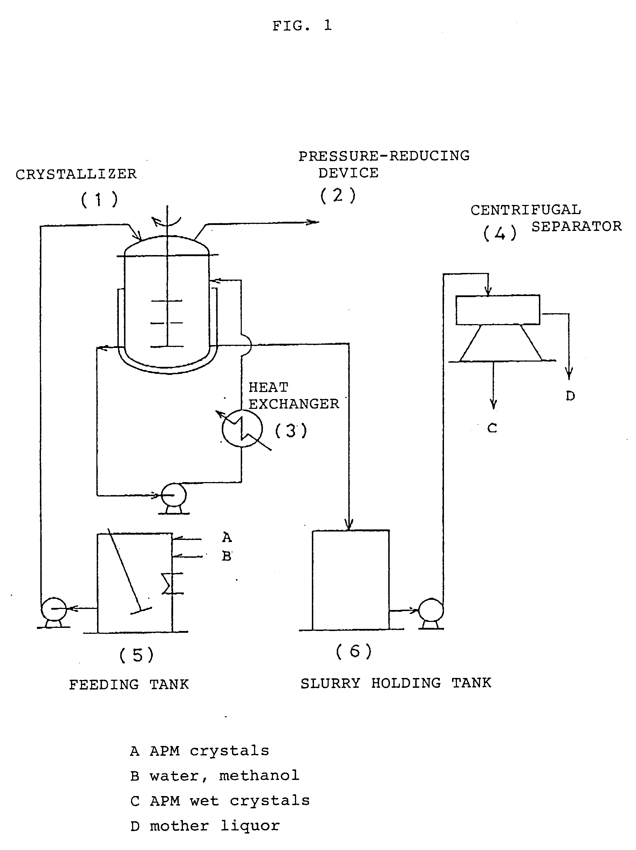

Figure 1 shows a diagram of a crystallizer equipment, which can be used in the present invention and was used in Example 1.

## DETAILED DESCRIPTION OF THE PREFERRED EMBODIMENTS

A crystallization operation is generally carried out such that, per unit time, a vaporizing amount of the solvent (water) per vaporizing area of the concentrated solution (or slurry) is about 1,000 kg/$m^2 \cdot$h for the crystallization of ordinary substances other than $\alpha$-APM. For crystallization of $\alpha$-APM, however, it has been discovered that a substantial bubbling of the slurry with vaporization occurs under conventional vaporization conditions. This bubbling is especially pronounced when the starting solution is fed to the crystallizer so that the depth of the solution in the crystallizer is kept constant. It is difficult to continue the operation of the equipment under these conditions. It is particularly preferred to control the vaporizing rate to a surprisingly low rate of 40 kg/$m^2 \cdot$h or less, more preferably 20 kg/$m^2 \cdot$h or less, so that a long and stable operation is possible.

A higher upper limit for the vaporizing rate is possible when using a mixed solvent of water and a lower alcohol, such as methanol or ethanol, than when using water alone. Further, when using a mixed solvent, equipment productivity is improved as the solubility of $\alpha$-APM in the mixed solvent over 40°C is higher than in water alone, and thus the crystals obtained have larger diameters and good solid-liquid separability. As the apparatus for the present invention, any industrially employable existing or widely-used crystallizer, such as a general stirring crystallizer, a DTB (Draft Tube Battled) crystallizer, a crystal-Oslo crystallizer or modifications thereof may be used, so long as they have an enclosed structure capable of withstanding the reduced pressure used in the present method. The pressure in the crystallizer is kept at 13 kPa (100 Torr) or less, preferably, 6.5 kPa (50 Torr) or less.

Crystallizers are available without heat transfer surfaces for cooling. It is preferable to equip such crystallizers with a heat transfer means such as coils, jackets and so on, or with an external heat exchanger whereby the solution can be heated by circulating a part of the $\alpha$-APM solution through the heat transfer means. Heating the $\alpha$-APM solution is preferable when the temperature of the solution becomes too low. When the temperature of the solution becomes too low, the vaporization rate is lowered, thereby decreasing the efficiency of the present process. Although the temperature of operation during the present process depends on the operation pressure, it is preferable to keep the temperature of the solution at 20°C or lower (the lower limit is the freezing point of the solvent) to obtain maximum stability of the process and high yield of $\alpha$-APM.

The starting $\alpha$-APM solution may be fed to the crystallizer continuously, batchwise or as a combination of batch and continuous operation. It is preferable in view of equipment productivity and yield to feed the $\alpha$-APM solution continuously keeping the depth of the solution constant. In this case, the yield per equipment volume can be increased by simultaneously discharging the concentrated solution or slurry (continuous vaporization operation).

Since the bubbling is remarkable as mentioned above, especially when using water as a solvent, it is necessary to control the vaporizing rate to 40 kg/$m^2 \cdot$h or less, preferably 20 kg/$m^2 \cdot$h or less, or to use an aqueous mixed solvent containing a lower alcohol, preferably a $C_{1-6}$ alkyl alcohol. Methanol and ethanol are preferably used as the lower alcohol. Methanol is the most preferred lower alcohol since $\alpha$-APM can undergo ester exchange reactions under some conditions reducing the overall yield of the process. The solid-liquid separability of the crystals deteriorates if the alcohol concentration is too high. Therefore, the alcohol concentration is preferably in the range of from 10 - 60% by volume of the solvent.

The concentration of $\alpha$-APM in the starting solution is preferably a saturated solution in the solvent used at 30°C or higher, e.g. the solubility of $\alpha$-APM at 30°C is 1-3 g/dl, since a dilute solution requires greater concentration to effect precipitation. The solubility of $\alpha$-APM increases at higher temperatures, however, decomposition of $\alpha$-APM simultaneously occurs, so, the maximum concentration of the starting solution should be the concentration of a saturated solution at 80°C, for example. A preferred range of the concentration of $\alpha$-APM in the starting solution is 2-15 %, more preferably 3-10 %.

The present method of crystallizing $\alpha$-APM overcomes deposition problems during crystallization. The use of water or an aqueous solvent containing a lower alcohol in the present process substantially improves the solid-liquid separability of $\alpha$-APM crystals and also noticeably improves the yield of crystals. The present method has practical utility due to its simplicity and the use of conventional equipment.

Other features of the invention will become apparent in the course of the following description of exemplary embodiments which are given for illustration of the invention and are not intended to be limiting thereof.

EXAMPLES

The test for evaluating the filterability of $\alpha$-APM crystals obtained in the Examples is described below.

Method of Measuring Filtration Specific Cake Resistance

One liter of a sample to be tested was sampled and filtered through a top-feed suction filter system (leaf tester). The pressure difference during filtration was 70 mmHg, which was kept constant throughout the period of filtration. From the start of filtration, the amount of the filtrate V(ml) was measured at regular intervals and plotted on a graph having the amount of the filtrate as the horizontal axis and the value $\theta$/V, obtained by dividing the time of filtration $\theta$ (s) by the amount of the filtrate, as the vertical axis. The slope of the line K(s/ml$^2$) was obtained by the least square method. The value C', obtained by dividing the total amount (g) of the crystals in the slurry by the total amount (cm$^3$) of the liquid in the slurry, was used in the following equation, where filtration area A is 93 (cm$^3$) and the viscosity $\mu$ of the filtrate is 0.0135 (g/cm.s). The specific cake resistance $\alpha$ (m/kg) thus calculated is a measure of the filterability of the sample. Samples having a lower value $\alpha$ are more easily filtered.

Equation for Specific Cake Resistance

$$\alpha = 20.K.A^2.PT/\mu.C' \text{ (m/kg)}$$

where $\alpha$ is the specific cake resistance (m/kg) of the filtered cake;

$\mu$ is the viscosity of the filtrate (g/cm.s) ;

PT is the pressure difference (dyne/cm$^2$) of the filtered cake and the filtration device = $\Delta$ P-(mmHg) x 1333.22;

A is the filtration area (cm$^2$); and

C' is the weight of the crystals per unit volume of the liquid component in the slurry (g/ml) = dry cake weight (g)/(wet cake weight (g) - dry cake weight (g) + amount of final filtrate (ml)).

Example 1:

A pilot plant system having the configuration shown in Fig. 1 was used. An aqueous $\alpha$-APM solution having an $\alpha$-APM concentration of 4.5 wt% and a liquid temperature of 65°C was fed into the 200-liter jacketed crystallizer (1), the inner pressure in the crystallizer was kept at from 8 to 10 Torr by an external pressure-reducing device (2), and the liquid temperature in the crystallizer was kept at 10°C by the latent heat of vaporization without cooling or heating. The vaporizing rate of the water solvent was 40 kg/m$^2$•h, and the slurry was discharged simultaneously so that the solution level in the tank was 50 %. The circulating speed of the solution was from 3 to 4 m$^3$/h. The operation was continued for 24 hours, whereupon no rapid foaming occurred during the operation and the slurry had good fluidity. The specific resistivity value of the slurry obtained was 7 x 10$^{10}$ (m/kg). After being filtrated with a small centrifuge followed by dewatering for 15 minutes, the crystals had a moisture content of 65 %.

Example 2:

The same experiment as that in Example 1 was repeated, except that the vaporizing rate of the water solvent was varied to 20 kg/m$^2$•h. The operation was continued for 24 hours, whereupon no rapid bubbling or foaming occurred during the operation and the slurry had good fluidity. The specific cake resistance value of the slurry obtained was 5.0 x 10$^{10}$ m/kg. After dewatering for 15 minutes, the crystals had a moisture content of 62.1%.

Comparative Example 1

The same operation as Example 1 was carried out except that a vaporizing rate of 100 kg/m$^2$•h was used. Just after the start of the operation the surface of the solution rose about one meter and it became difficult to continue the operation. The operation was stopped after 5 hours.

Example 3:

The same apparatus as used in Example 1 was used. An aqueous 30% methanol solution of $\alpha$-APM having an $\alpha$-APM concentration of 4.3 wt% and a liquid temperature of 50°C was fed into the crystallizer (1) at a feed speed of 188 liter/h (average),the inner pressure in the crystallizer was kept at about 4 MPa (30 Torr) using the external pressure-reducing device (2), and the liquid temperature in the crystallizer was kept at 20°C. The vaporizing rate of the solvent was 53 kg/m$^2$•h, and the slurry was discharged at a rate of 176 liter/h (average) to keep the slurry level constant. The operation was continued for 24 hours, whereupon no rapid foaming occurred during the operation and the slurry had good fluidity. The specific cake resistance value of the slurry obtained was 8.6 x 10$^9$ m/kg. After dewatering for 15 minutes with a centrifuge, the crystals had a moisture content (loss on dry) of 44 %.

Example 4:

The same apparatus as used in Example 1 was used. An aqueous 30% methanol solution of $\alpha$-APM having an $\alpha$-APM concentration of 3.7 wt% and a liquid temperature of 20°C was fed into the crystallizer (1) at a feed speed of 237 liter/h (average), and the inner pressure in the crystallizer was kept at about 4 kPa (30 Torr) using the external pressure-reducing device (2). A part of the $\alpha$-APM solution was removed from the crystallizer and heated with an external heat exchanger (3), to keep the liquid temperature in the crystallier at 20°C.

The concentration ratio was controlled to 0.8. The vaporizing rate of the solvent was 160 kg/m$^2$•h, and the circulating speed of the solution was 0.4 m$^3$/h. The operation was continued for 24 hours, whereupon no rapid foaming occurred during the operation and the slurry had good fluidity. The specific cake resistance value of the slurry obtained was 2.8 x 10$^9$ m/kg. After dewatering for 15 minutes with a centrifuge, the crystals had a moisture content (loss on dry) of 37.5%.

Obviously, numerous modifications and variations of the present invention are possible in light of the above teachings. It is therefore to be understood that within the scope of the appended claims, the invention may be practiced otherwise than as specifically described herein.

## Claims

1. A method of crystallizing $\alpha$-L-aspartyl-L-phenylalanine methyl ester from an aqueous solvent solution thereof, comprising:
   crystallizing $\alpha$-L-aspartyl-L-phenylalanine methyl ester in a crystallizer, wherein the pressure in the crystallizer is maintained at or below atmospheric pressure to vaporize said solvent and cooling said solution by the latent heat of vaporization.

2. The method of Claim 1, wherein said pressure in the crystallizer is 13 kPa (100 Torr) or less, preferably 6.5 kPa (50 Torr) or less.

3. The method of Claim 1 or 2, wherein the temperature of said solution is 20°C or less during said crystallizing step.

4. The method of any of the Claims 1 to 3, further comprising heating said solution indirectly with a heat exchange means during said crystallizing step.

5. The method of any of the Claims 1 to 4, wherein said aqueous solvent solution is continuously fed to the crystallizer, maintaining a substantially uniform depth of solution in the crystallizer to form a concentrated slurry.

6. The method of Claim 5, further comprising continuously discharging said concentrated slurry from said crystallizer.

7. The method of any of the Claims 1 to 6, wherein when water is used as a solvent the amount of the solvent vaporized per vaporization area of the said solution per unit time is 40 kg/m$^2$•h or less, preferably 20 kg/m$^2$•h or less.

8. The method of any of the Claims 1 to 7, wherein said solvent is a mixed solvent comprising water and a lower alcohol.

9. The method of Claim 8, wherein said solution is concentrated by evaporating a part of said solvent.

10. The method of Claim 9, wherein said lower alcohol is methanol.

11. The method of Claim 9, wherein the methanol concentration in said solution is from 10 to 60 % by volume.

## Patentansprüche

1. Verfahren zur Kristallisation von $\alpha$-L-Aspartyl-L-phenylalanin-methylester aus dessen Lösung in einem wäßrigen Lösungsmittel, welches die Kristallisation von $\alpha$-L-Aspartyl-L-phenylala-nin-methylesterin einem Kristallisationsgefäß umfaßt, wobei der Druck in dem Kristallisationsgefäß bei oder unterhalb von Atmosphärendruck gehalten wird, so daß das Lösungsmittel verdampft und die Lösung durch die latente Verdampfungswärme gekühlt wird.

2. Verfahren nach Anspruch 1, wobei der Druck in dem Kristallisationsgefäß 13 MPa (100 Torr) oder weniger, vorzugsweise 6,5 kPa (50 Torr) oder weniger, beträgt.

3. Verfahren nach Anspruch 1 oder 2, wobei die Temperatur der Lösung während der Kristallisationsstufe 20°C oder weniger beträgt.

4. Verfahren nach einem der Ansprüche 1 bis 3, welches außerdem das indirekte Erwärmen der Lösung mit Hilfe einer Wärmetauschvorrichtung während der Kristallisationsstufe umfaßt.

5. Verfahren nach einem der Ansprüche 1 bis 4, wobei die Lösung in dem wäßrigen Lösungsmittel dem Kristallisationsgefäß kontinuierlich zugeführt wird und eine im wesentlichen gleichmäßige Tiefe der Lösung in dem Kristallisationsgefäß aufrecht erhalten wird, um eine konzentrierte Aufschlämmung auszubilden.

6. Verfahren nach Anspruch 5, welches außerdem die kontinuierliche Entnahme der konzentrierten Aufschlämmung aus dem Kristallisationsgefäß umfaßt.

7. Verfahren nach einem der Ansprüche 1 bis 6, wobei dann, wenn Wasser als Lösungsmittel verwendet wird, die Menge des pro Verdampfungsfläche der Lösung in der Zeiteinheit verdampften Lösungsmittels 40 kg/m²·h oder weniger, oder vorzugsweise 20 kg/m²·h oder weniger, beträgt.

8. Verfahren nach einem der Ansprüche 1 bis 7, wobei das Lösungsmittel ein Wasser und einen niederen Alkohol enthaltendes Mischlösungsmittel ist.

9. Verfahren nach Anspruch 8, wobei die Lösung durch Verdampfen eines Teils des Lösungsmittels konzentriert wird.

10. Verfahren nach Anspruch 9, wobei der niedere Alkohol Methanol ist.

11. Verfahren nach Anspruch 9, wobei die Methanolkonzentration in der Lösung 10 bis 60 Vol.% beträgt.

## Revendications

1. Procédé de cristallisation d'ester méthylique d'α-L-aspartyl-L-phénylalanine à partir de sa solution aqueuse de solvant comprenant :

la cristallisation d'ester méthylique d'α-L-aspartyl-L-phénylalanine dans un cristallisoir, dans laquelle la pression du cristallisoir est maintenue à une pression inférieure ou égale à la pression atmosphérique pour vaporiser ledit solvant, ainsi que le refroidissement de ladite solution par la chaleur latente de vaporisation.

2. Procédé selon la revendication 1, dans lequel ladite pression du cristallisoir est inférieure ou égale à 13 MPa (100 Torr), de préférence inférieure ou égale à 6,5 MPa (50 Torr).

3. Procédé selon la revendication 1 ou 2, dans lequel la température de ladite solution est inférieure ou égale à 20°C lors de ladite étape de cristallisation.

4. Procédé selon l'une quelconque des revendications 1 à 3, comprenant de plus le chauffage de ladite solution indirectement par un dispositif d'échange thermique lors de ladite étape de cristallisation.

5. Procédé selon l'une quelconque des revendications 1 à 4, dans lequel on introduit continuellement ladite solution aqueuse de solvant dans le cristallisoir, maintenant une hauteur sensiblement uniforme de solution dans le cristallisoir pour former une suspension épaisse concentrée.

6. Procédé selon la revendication 5, comprenant de plus un déversement en continu de ladite suspension épaisse concentrée à partir dudit cristallisoir.

7. Procédé selon l'une quelconque des revendications 1 à 6, dans lequel, lorsqu'on utilise de l'eau en tant que solvant, la quantité de solvant vaporisé par surface de vaporisation de ladite solution par unité de temps est inférieure ou égale à 40 kg/m².h, de préférence inférieure ou égale à 20 kg/m².h.

8. Procédé selon l'une quelconque des revendications 1 à 7, dans lequel ledit solvant est un solvant mixte comprenant de l'eau et un alcool inférieur.

9. Procédé selon la revendication 8, dans lequel on concentre ladite solution par évaporation d'une partie dudit solvant.

10. Procédé selon la revendication 9, dans lequel ledit alcool inférieur est le méthanol.

11. Procédé selon la revendication 9, dans lequel la concentration en méthanol de ladite solution est comprise entre 10 et 60 % en volume.

FIG. 1

CRYSTALLIZER (1)

PRESSURE-REDUCING DEVICE (2)

CENTRIFUGAL (4) SEPARATOR

HEAT EXCHANGER (3)

(5) FEEDING TANK

(6) SLURRY HOLDING TANK

A APM crystals
B water, methanol
C APM wet crystals
D mother liquor